# EUROPEAN PATENT APPLICATION

(11) **EP 2 921 179 A1**
(43) Date of publication of application: **23.09.2015**
(21) Application number: 14382093.4
(22) Date of filing: 17.03.2014
(51) Int. Cl.: A61K 41/00, A61K 9/50, A61K 9/51, A61K 31/00

(54) **Micellar nanoparticles containing antitumoral glycosides**

(71) Applicant: Fundación Centro Nacional de Investigaciones Cardiovasculares Carlos III (CNIC), 28029 Madrid (ES); Consejo Superior De Investigaciones Científicas, 28006 Madrid (ES); CIBER Centro de Investigación Biomédica en Red, 28029 Madrid (ES)
(72) Inventor: Herranz Rabanal, Fernando, E-28029 Madrid (ES); Ruiz-Cabello Osuna, Jesús, E-28029 Madrid (ES); Fernandez-Mayoralas Álvarez, Alfonso, E-28006 Madrid (ES); Nieto Sampedro, Manuel, E-28002 Madrid (ES); Amaury Groult, Hugo, E-28029 Madrid (ES)
(74) Representative: ABG Patentes, S.L.

(57) **Abstract**

The present invention is related to micelles which comprise nanoparticles having a core selected from the group consisting of iron oxide, UCNP and a metal, and a hydrophobic outer layer, and a coating thereon comprising glycoside derivatives, a process for their preparation and uses thereof.

## Description

### FIELD OF THE INVENTION

The present invention relates to micelles comprising nanoparticles and a coating comprising glycoside derivatives, the process of obtaining of said micelles, as well as the use thereof in the treatment of cancer.

### BACKGROUND OF THE INVENTION

Glycoside derivatives conjugated to a hydrophobic moiety have been described as having antitumor activity in different tumoral cell lines such as glioma, lung carcinoma and melanoma [García-Álvarez I. et al., J. Med. Chem. 2011, 54, 6949-6955; García-Álvarez I. et al., J. Med. Chem. 2007, 50, 364-373; and Fernández-Mayoralas A. et al., J. Med. Chem. 2003, 46, 1286-1288]. However, these compounds generally show poor water solubility, rendering them less suitable as therapeutic agents.

Functionalized magnetic nanoparticles have been described for delivery of water insoluble anticancer agents.

Jain T.K. et al. [Molecular Pharmaceutics, 2005, 2, 193-205] discloses water dispersible oleic acid-Pluronic-coated iron oxide magnetic nanoparticle formulations as a universal drug carrier system for systemic administration of water insoluble drugs, such as water insoluble anticancer agents. The Pluronic coating provides water solubility to the iron oxide nanoparticles, which may be then loaded with the water insoluble drug. The hydrophobic drug is partitioned in the oleic acid shell surrounding the iron oxide nanoparticles. However, the presence of the Pluronic coating is necessary for achieving the solubilization of the drug.

Lee C.M. et al [Bioconjugate Chemistry, 2011, 22, 186-192] discloses oleic acid decorated iron oxide nanoparticles encapsulated with oleic acid conjugated chitosan, which tend to accumulate in tumors and form micelles having a hydrophobic core and hydrophilic shell structure in aqueous solutions. Oleyl-chitosan self-aggregates in a structure comprising a hydrophobic core, wherein the iron oxide nanoparticles and the hydrophobic drug may be loaded, and a hydrophilic shell. However, the presence of the oleyl-chitosan coating is necessary for achieving the solubilization of the drug.

In addition, magnetic nanoparticles functionalized with glycoside derivatives and therapeutic agents have been described in the prior art for enhancing tumor accumulation of the therapeutic agent without disclosing any improvement of the water solubility of said therapeutic agents.

Document WO 2009/123734 discloses magnetic nanoparticles functionalized with 2-deoxyglucose, which may additionally comprise a therapeutic agent for the treatment of several diseases. Said 2-deoxyglucose functionalized nanoparticles may exhibit a greater metabolic uptake into cancerous tissue.

Document WO 2011/107290 discloses magnetic nanoparticles functionalized by a ligand that is adequate for internalization into cancerous cells, such as a monosaccharide (for example glucose), dextrane, polyvinylpyrrolidone, polyvinylalcohol, oleic acid or combinations thereof. Said functionalized nanoparticles may be used for cancer diagnosis and treatment purposes.

Document WO 2011/095661 discloses magnetic nanoparticles in combination with an active ingredient for the treatment of cancer. Due to the magnetic nature of the nanoparticles, they allow the concentration of the active ingredient in specific locations of the body once they have been administered to the patient.

Even though solubilization of water insoluble drugs has been described in the prior art in systems comprising nanoparticles, the presence of an amphiphilic polymer is necessary for achieving this goal. Moreover, in the prior art, glycosides are either covalently attached to the coating (in this case they are participating to the solubilization of the nanoparticles probe but do not act as drug) or encapsulated as a drug in a micelle, but then they do not participate in the solubilization. Thus, there remains a need to develop a simple system that allows the solubilization of glycoside derivatives having anticancer activity. The authors of the present invention have solved this need by providing a micelle comprising a nanoparticle core and glycoside derivative coating, which are bonded by hydrophobic interactions. In some cases, the use of such a system provides an increase in the antitumoral activity of the glycoside derivatives.

### SUMMARY OF THE INVENTION

In a first aspect, the present invention relates to a micelle comprising:
a) one or more nanoparticles having a core selected from the group consisting of iron oxide, UCNP and metals, and an outer layer comprising a first member of a hydrophobic interaction pair; and
b) a coating comprising glycoside derivatives of formula (I) 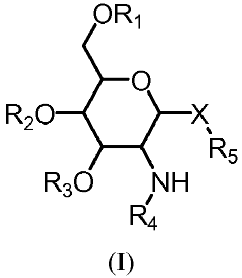 wherein
   R₁ is selected from the group consisting of H; C₁-C₆ alkyl optionally substituted with one or more substituents selected from the group consisting of C₁-C₃ alkyl, hydroxyl-C₁-C₃ alkyl, and hydroxyl; C₁-C₆ acyl; and SO₃M wherein M is selected from the group consisting of hydrogen, alkali metal, ammonium and quaternary amine;
   R₂ and R₃ are independently selected from the group consisting of H and C₁-C₆ acyl;
   R₄ is selected from the group consisting of H and C₁-C₃ acyl optionally substituted with one or more halogen atoms;
   R₅ is a second member of the hydrophobic interaction pair; and
   X is selected from the group consisting of O and S;
   or a stereoisomer thereof;
wherein the coating is associated to the nanoparticle by means of hydrophobic interactions between the first member of the hydrophobic interaction pair of the outer layer of the nanoparticles and the R₅ groups in the coating.

In a second aspect, the present invention relates to a process for the preparation of a micelle as defined in the first aspect which comprises:
a) providing one or more nanoparticles comprising a core and an outer layer as defined in any the first aspect;
b) providing glycoside derivatives of formula (I) as defined in any the first aspect; and
c) mixing the nanoparticles of step a) and the glycoside derivatives of step b) under conditions adequate for the formation of hydrophobic interactions between the first member of the hydrophobic interaction pair of the outer layer of the nanoparticles and the R₅ groups in the glycoside derivatives.

In a third aspect, the present invention relates to a micelle as defined in the first aspect for use in medicine.

In a fourth aspect, the present invention relates to a micelle as defined in the first aspect for use in the treatment and/or prevention of cancer.

### DESCRIPTION OF THE FIGURES

**Figure 1** shows TEM pictures of the micelles of the invention obtained in Example 3 having as glycoside derivative GC22 (1), TFAGC22 (2) and IG20 (3).
**Figure 2** shows the Fourier transformed infrared spectra of the micelles of the invention obtained in Example 3 having as glycoside derivative GC22 (1), TFAGC22 (2) and IG20 (3).
**Figures 3A-3D** shows *in vitro* bioactivity of the micelles of the invention determined in Example 4 against C6 (rat glioma) cell line.
**Figures 4A-4D** shows *in vitro* bioactivity of the micelles of the invention determined in Example 4 against A549 (adenocarcinoma human lung) cell line.
**Figure 5** shows the kinetic of the elimination of GC22-NP micelle in liver after intravenous administration determined in Example 5. Negative signal enhancement is normalized to the baseline image of the liver and calculated on a selected set of regions of interest (ROIs).

### DESCRIPTION OF THE INVENTION

### Micelles comprising nanoparticles

In the first aspect, the present invention relates to a micelle that comprises a) one or more nanoparticles having a core selected from the group consisting of iron oxide, UCNP and metals, and an outer layer comprising a first member of a hydrophobic interaction pair and b) a coating comprising glycoside derivatives of formula (I), as defined above.

In the context of the present invention, the term "micelle" refers to an assembly between one or more nanoparticles and a coating as defined above. Preferably the hydrodynamic size of the micelle ranges from 1 to 1000 nm, preferably from 10 nm to 250 nm, even more preferably between 10 nm and 100 nm, most preferably between 10 nm and 80 nm.

In the context of the present invention, "nanoparticle" must be understood as a particle, preferably a spherical particle, with a hydrodynamic size ranging from 1 to 1000 nm, preferably from 1 nm to 200 nm, even more preferably between 3nm and 50 nm, still more preferably between 5 nm and 30 nm, and most preferably between 20 nm and 60 nm.

The core of the nanoparticles is selected from the group consisting of iron oxide, UCNP and metals, preferably the core is an iron oxide core.

The type of iron oxide can be selected from any of the common iron oxides in the nanoparticle field and that are known by the person skilled in the art, such as for example, Fe₃O₄ (magnetite), α-Fe₂O₃ (hematite), γ-Fe₂O₃ (maghemite), FeO (wüstite), ε-Fe₂O₃ and β-Fe₂O₃, among others. In a preferred embodiment of the invention, the iron oxide is selected from Fe₃O₄ and γ-Fe₂O₃.

The term "up-converting nanophosphor" or "UCNP" refers to rare-earth nanoparticles that have the capability to turn near infrared (NIR) light energy into visible light or higher energy NIR. Examples of UCNP are those the core of which consists of NaXF₄ or XF₃ matrix where X may be any of Y, Gd, Lu, Yb or other lanthanides and doped with Er,Tm,Yb, Gd, Lu or other lanthanides. Common examples are NaYF₄ or NaGdF₄ doped with Yb, Er, or Tm. An inorganic layer made of the same matrix NaXF₄ or XF₃ previously described with or without the dopants may also be added around the core. This inorganic layer above all improves fluorescence properties. For example UNCPs are preferably NaGdF₄Yb_{25%},Tm_{0.5%}@NaGdF₄, i.e., a core consisting of NaGdF₄ doped with Yb and Tm and surrounded by a layer of NaGdF₄.

The type of metal can be selected from any of the common metal cores used in the field and that are known by the person skilled in the art, such as silver, gold, ruthenium, platinum, copper, and palladium. Preferably the metal nanoparticle core is selected from the group consisting of silver and gold.

The core of the nanoparticle is surrounded by an outer layer that comprises a first member of a hydrophobic interaction pair.

The term "outer layer" refers to the layer or shell that surrounds the nanoparticle core. In the context of the present invention, the outer layer has an organic composition, i.e. it does not consist of inorganic components.

The term "hydrophobic interaction pair" refers to two members, a first member and a second member, that are capable of forming a complex between the outer layer of the nanoparticles and the coating that comprises glycoside derivatives of formula (I) through hydrophobic interactions of said first and second members. Preferably, said first member is a fatty acid. Preferably said second member is a fatty acid radical. More preferably, said first member is a fatty acid and said second member is a fatty acid radical.

A hydrophobic interaction also known as van der Waals interaction can be determined by methods known in the art such as by common techniques for the assessment of micelle formation (for example transmission electron microscopy (TEM) and dynamic light scattering (DLS). Other methods include observing that no re-dispersion is produced when the aqueous micellar solution if mixed of an organic solvent selected from the group consisting of dichloromethane, chloroform, hexane, heptane, toluene, preferably in a 1:1 volume proportion, although other proportions may be used.

The term "fatty acid" refers to a long chain alkyl or alkenyl radical, such as C₈-C₃₀ alkyl or C₈-C₃₀ alkenyl, preferably C₈-C₃₀ alkenyl, bound to a -COOH moiety. Examples of alkenyl fatty acids are oleic acid (*cis-*CH₃(CH₂)₇CH=CH(CH₂)₇-COOH), myristoleic acid (*cis*-CH₃(CH₂)₃CH=CH(CH₂)₇-COOH), palmitoleic acid (*cis-*CH₃(CH₂)₅CH=CH(CH₂)₇-COOH), sapienic acid (*cis-*CH₃(CH₂)₈CH=CH(CH₂)₄-COOH), elaidic acid (*trans*-CH₃(CH₂)₇CH=CH(CH₂)₇-COOH), vaccenic acid (*trans-*CH₃(CH₂)₅CH=CH(CH₂)₉-COOH), linoleic acid (*cis,cis-*CH₃(CH₂)₄CH=CHCH₂CH=CH(CH₂)₇-COOH), linoleaidic acid (*trans,trans-*CH₃(CH₂)₄CH=CHCH₂CH=CH(CH₂)₇-COOH), linolenic acid (*cis,cis,cis-*CH₃CH₂CH=CHCH₂CH=CHCH₂CH=CH(CH₂)₇-COOH), arachidonic acid (*cis,cis,cis,cis*-CH₃(CH₂)₄CH=CHCH₂CH=CHCH₂CH=CHCH₂CH=CH(CH₂)₃-COOH), eicosapentanenoic acid (*cis,cis,cis,cis,cis-*CH₃CH₂CH=CHCH₂CH=CHCH₂CH=CHCH₂CH=CHCH₂CH=CH(CH₂)₃-COOH), erucic acid (*cis*-CH₃(CH₂)₇CH=CH(CH₂)₁₁-COOH), and docosahexaenoic acid (*cis,cis,cis,cis,cis,cis-*CH₃CH₂CH=CHCH₂CH=CHCH₂CH=CHCH₂CH=CHCH₂CH=CHCH₂CH=CH (CH₂)₂-COOH); and examples of alkyl fatty acids are caprylic acid (CH₃(CH₂)₆-COOH), capric acid (CH₃(CH₂)₈-COOH), lauric acid (CH₃(CH₂)₁₀-COOH), myristic acid (CH₃(CH₂)₁₂-COOH), palmitic acid (CH₃(CH₂)₁₄-COOH), stearic acid (CH₃(CH₂)₁₆-COOH), arachidic acid (CH₃(CH₂)₁₈-COOH), behenic acid (CH₃(CH₂)₂₀-COOH), lignoceric acid (CH₃(CH₂)₂₂-COOH) and cerotic acid (CH₃(CH₂)₂₄-COOH). Preferably, the fatty acid is a C₈-C₃₀ alkenyl-COOH fatty acid; more preferably a fatty acid is selected from the group consisting of oleic acid, myristoleic acid, palmitoleic acid and sapienic acid; still more preferably, the fatty acid is oleic acid.

The term "fatty acid radical" refers to a long chain alkyl or alkenyl radical, such as C₈-C₃₀ alkyl or C₈-C₃₀ alkenyl, preferably C₈-C₃₀ alkenyl, i.e. a fatty acid as defined above not containing the -COOH moiety. Examples of fatty acids akenyl radicals are oleyl (*cis*-CH₃(CH₂)₇CH=CH(CH₂)₇-), myristoleyl (*cis*-CH₃(CH₂)₃CH=CH(CH₂)₇-), palmitoleyl (*cis*-CH₃(CH₂)₅CH=CH(CH₂)₇-), sapienyl (*cis*-CH₃(CH₂)₈CH=CH(CH₂)₄-), elaidyl (*trans*-CH₃(CH₂)₇CH=CH(CH₂)₇-), vaccenyl (*trans*-CH₃(CH₂)₅CH=CH(CH₂)₉-), linoleyl (*cis*,*cis*-CH₃(CH₂)₄CH=CHCH₂CH=CH(CH₂)₇-), linoleaidyl (*trans,trans-*CH₃(CH₂)₄CH=CHCH₂CH=CH(CH₂)₇-), linolenyl (*cis,cis,cis-*CH₃CH₂CH=CHCH₂CH=CHCH₂CH=CH(CH₂)₇-), arachidonyl (*cis,cis,cis,cis-*CH₃(CH₂)₄CH=CHCH₂CH=CHCH₂CH=CHCH₂CH=CH(CH₂)₃-), eicosapentanenoyl (*cis,cis,cis,cis,cis-*CH₃CH₂CH=CHCH₂CH=CHCH₂CH=CHCH₂CH=CHCH₂CH=CH(CH₂)₃-), erucyl (*cis*-CH₃(CH₂)₇CH=CH(CH₂)₁₁-), and docosahexaenoyl (*cis,cis,cis,cis,cis,cis-*CH₃CH₂CH=CHCH₂CH=CHCH₂CH=CHCH₂CH=CHCH₂CH=CHCH₂CH=CH (CH₂)₂-); and examples of fatty acid alkyl radicals are caprylyl (CH₃(CH₂)₆-), octyl (CH₃(CH₂)₇-) capryl (CH₃(CH₂)₈-), lauryl (CH₃(CH₂)₁₀-), myristyl (CH₃(CH₂)₁₂-), palmityl (CH₃(CH₂)₁₄-), stearyl (CH₃(CH₂)1₆-), arachidyl (CH₃(CH₂)₁₈-), behenyl (CH₃(CH₂)₂₀-), lignoceryl (CH₃(CH₂)₂₂-) and cerotyl (CH₃(CH₂)₂₄-). Preferably, the fatty acid radical is a fatty acid is selected from the group consisting of oleyl, octyl, myristoleyl, palmitoleyl and sapienyl; more preferably, the fatty acid radical is selected from oleyl and octyl; even more preferably, the fatty acid radical is oleyl.

The term "long chain alkyl" refers to a linear, cyclic or branched hydrocarbon chain radical consisting of carbon and hydrogen atoms, not containing unsaturation, having 8 to 30, preferably 10 to 20 carbon atoms, and bound to the rest of the molecule by means of a single bond. The long chain alkyl groups can optionally be substituted with one or more substituents such as halogen, for example F, Cl, Br and I, carbonyl and nitro.

The term "long chain alkenyl" refers to a linear, cyclic or branched hydrocarbon chain radical consisting of carbon and hydrogen atoms, containing at least one double bond, having 8 to 30, preferably 10 to 21 carbon atoms, and bound to the rest of the molecule by means of a single bond. The long chain alkenyl groups can optionally be substituted with one or more substituents such as halogen, for example F, Cl, Br and I, carbonyl and nitro.

The term "first member of a hydrophobic interaction pair" refers to the member of the hydrophobic interaction pair that is present in the outer layer of the nanoparticles and comprises a polar head and a nonpolar tail. The polar head of the first member of the hydrophobic interaction pair, preferably a -COOH moiety, interacts ionically/covalently with the surface of the core of the nanoparticles, whereas the nonpolar tail of the first member of the hydrophobic interaction pair, preferably a fatty acid radical, interacts hydrophobically with the second member of the hydrophobic interaction pair, i.e. R₅ in the glycoside derivative of formula (I) as defined above. Preferably, the first member of the hydrophobic interaction pair is a fatty acid as defined above; even more preferably a C₈-C₃₀ alkenyl-COOH fatty acid as defined above; more preferably the first member of the hydrophobic interaction pair is selected from the group consisting of oleic acid, myristoleic acid, palmitoleic acid and sapienic acid; still more preferably, the first member of the hydrophobic interaction pair is oleic acid.

The term "second member of a hydrophobic interaction pair" refers to the member of the hydrophobic interaction pair that is present in the glycoside derivatives of formula (I) as substituent R₅. R₅ is a fatty acid radical as defined above, i.e. as C₈-C₃₀ alkyl or C₈-C₃₀ alkenyl, as defined above. Preferably the second member of the hydrophobic interaction pair is selected from the group consisting of oleyl, octyl, myristoleyl, palmitoleyl and sapienyl; more preferably, the second member of the hydrophobic interaction pair is selected from oleyl and octyl; even more preferably the second member of the hydrophobic interaction pair is oleyl.

The first and second member of the hydrophobic interaction pair may be the same or may be different. When the first and second members of the hydrophobic interaction pair are a fatty acid and a fatty acid radical, respectively, the "same" is to be understood as the fatty acid and radical derived from said fatty acid, for example oleic acid and oleyl. When the first and second members of the hydrophobic interaction pair are a fatty acid and a fatty acid radical, respectively, "different" is to be understood as the fatty acid and radical derived from another fatty acid, for example oleic acid and octyl.

In a particular embodiment, the first member of the hydrophobic interaction pair is a C₈-C₃₀ alkenyl-COOH fatty acid as defined above and the second member of the hydrophobic interaction pair is selected from the group consisting of oleyl, octyl, myristoleyl, palmitoleyl and sapienyl; more preferably the first member of the hydrophobic interaction pair is selected from the group consisting of oleic acid, myristoleic acid, palmitoleic acid and sapienic acid, and the second member of the hydrophobic interaction pair is selected from the group consisting of oleyl, octyl, myristoleyl, palmitoleyl and sapienyl; still more preferably, the first member of the hydrophobic interaction pair is oleic acid and the second member of the hydrophobic interaction pair (R₅) is oleyl or octyl; even more preferably, the first member of the hydrophobic interaction pair is oleic acid and the second member of the hydrophobic interaction pair (R₅) is oleyl.

A micelle is formed due to the hydrophobic interaction of the first and second members of the hydrophobic interaction pair. Thus, said micelle comprises the core of the nanoparticle surrounded by an outer layer that comprises the first member of the hydrophobic interaction pair, which is hydrophobically bound (i.e. by van der Waals interaction) to the second member of the hydrophobic interaction pair, thus, forming a coating of glycoside derivatives of formula (I) on the surface of the outer layer of the nanoparticles. Thus, the micelle comprises, from inwards to outwards, the nanoparticle core, an outer layer surrounding said nanoparticle core, and a coating comprising the glycoside derivatives of formula (I). Since the group R₅ of the glycoside derivative of formula (I), i.e. the second member of the hydrophobic interaction pair, interacts with the outer layer of the nanoparticles, the remaining part of the glycoside derivative of formula (I) (the part of the molecule that is not R₅) forms the outer surface of the micelle and presents a hydrophilic character.

The glycoside derivative of formula (I), as defined above, forms the coating of the micelle.

Any compound referred to herein is intended to represent such specific compound as well as certain variations or forms. In particular, compounds referred to herein may have asymmetric centres and therefore exist in different enantiomeric forms. All optical isomers and stereoisomers of the compounds referred to herein, and mixtures thereof, are considered within the scope of the present invention. Thus any given compound referred to herein is intended to represent any "stereoisomer", i.e. any one of a racemate, one or more enantiomeric forms, one or more diastereomeric forms, one or more atropisomeric forms, and mixtures thereof. Particularly, the compounds of the present invention represented by the above described formula I may include enantiomers depending on their asymmetry or diastereoisomers. Stereoisomerism about the double bond is also possible, therefore in some cases the molecule could exist as (E)-isomer or (Z)-isomer. If the molecule contains several double bonds, each double bond will have its own stereoisomerism, that could be the same or different than the stereoisomerism of the other double bonds of the molecule. The single isomers and mixtures of isomers fall within the scope of the present invention.

R₁ is selected from the group consisting of H; C₁-C₆ alkyl optionally substituted with one or more substituents independently selected from the group consisting of C₁-C₃ alkyl, hydroxyl-C₁-C₃ alkyl, and hydroxyl; C₁-C₆ acyl; and SO₃M wherein M is selected from the group consisting of hydrogen, alkali metal, ammonium and quaternary amine. Preferably, R₁ is selected from the group consisting of H, SO₃M wherein M is selected from the group consisting of hydrogen, alkali metal, ammonium and quaternary amine, and C₁-C₃ alkyl optionally substituted with one or more substituents independently selected from the group consisting of hydroxyl-C₁-C₃ alkyl and hydroxyl. More preferably, R₁ is selected from the group consisting of H, SO₃M wherein M is selected from the group consisting of hydrogen, Na, K and ammonium, and C₁-C₃ alkyl optionally substituted with one or more substituents independently selected from the group consisting of hydroxyl-C₁-C₃ alkyl and hydroxyl. Still more preferably, R₁ is selected from the group consisting of H, SO₃K, and propyl substituted with one, two or three substituents independently selected from methylhydroxyl and hydroxyl.

The term "acyl" refers to an alkyl-C(=O)- moiety. The number of carbon atoms in the acyl group refers to the number of carbon atoms present in the alkyl group, i.e. it does not include the carbon of the C=O moiety. Thus, a C₁ acyl refers to acetyl (CH₃C(=O)).

The term "alkyl" refers to a straight or branched hydrocarbon chain radical consisting of carbon and hydrogen atoms, containing no saturation, having one to six carbon atoms, preferably one to three carbon atoms, and which is attached to the rest of the molecule by a single bond. Examples of alkyl are methyl, ethyl, n-propyl, isopropyl, n-butyl, tert-butyl, sec-butyl, n-pentyl and n-hexyl.

The term "hydroxyl" refers to OH.

The term "one or more substituents" refers to 1, 2, 3, 4, 5 or 6 substituents, preferably 1, 2 or 3 substituents.

Examples of "alkali metal" are Li, Na, K, Rb, Cs and Fr, preferably Na and K, more preferably K.

The term "quaternary amine" refers to a group of formula N⁺RₐR_{b}R_{c}R_{d}, wherein Rₐ, R_{b}, R_{c} and R_{d} are independently selected from the group consisting of C₁-C₆ alkyl, C₂-C₆ alkenyl, C₆-C₁₄ aryl, C₆-C₁₄ aryl-C₁-C₃ alkylene, said alkyl and aryl being optionally substituted with one, two, three or four substituents independently selected form the group consisting of OH, C₁-C₆ alkyl and C₁-C₆ alkoxy. Examples of quaternary amines are tetramethylammonium, tetraethylammonium, ethyltrimethylammonium, benzyltriethylammonium, choline, among others.

The term "alkenyl" refers to a straight or branched hydrocarbon chain radical consisting of carbon and hydrogen atoms, containing one or two double bonds, having two to six carbon atoms, preferably two to three carbon atoms, and which is attached to the rest of the molecule by a single bond. Examples of alkenyl are ethenyl, propenyl, allyl, butenyl, 1-methyl-2-buten-1-yl, and the like.

The term "alkylene" as employed herein alone or as part of another group designates a linear or branched saturated divalent hydrocarbon chain containing from of from one to three carbon atoms. Examples of alkylene are methylene (-CH₂-), ethylene (-CH₂CH₂-) and trimethylene (-CH₂CH₂CH₂-).

The term "alkoxy" as employed herein alone or as a part of another group designates an alkyl group as defined above linked through oxygen. Examples of alkoxy include methoxy, ethoxy, isopropoxy, tertbutoxy, and the like.

The term "aryl" means a mono-, bi- or tricyclic aromatic hydrocarbon group, containing the number of atoms indicated in each case, which is comprised between six and fourteen carbon atoms in the ring portion, wherein the monocyclic ring is aromatic, and at least one of the rings in the bi- or tricyclic ring is aromatic. Representative examples include phenyl, naphthyl, phenanthryl, indenyl, indanyl, and the like, preferably phenyl.

When R₁ is SO₃M and M is alkali metal, ammonium or quaternary amine, a salt is formed between the corresponding SO₃- group and the corresponding metal cation (Li⁺, Na⁺, K⁺, Rb⁺, Cs⁺ or Fr⁺, preferably Na⁺ or K⁺, more preferably K⁺), ammonium (NH₄⁺) or quaternary amine (N⁺RₐR_{b}R_{c}R_{d}).

R₂ and R₃ are independently selected from the group consisting of H and C₁-C₆ acyl, preferably H.

R₄ is selected from the group consisting of and C₁-C₃ acyl optionally substituted with one or more halogen atoms. Preferably R₄ is selected from the group consisting of H, acetyl and trifluoroacetyl.

R₅ is a second member of the hydrophobic interaction pair, as defined above. Preferably R₅ is selected from C₈-C₃₀ alkyl or C₈-C₃₀ alkenyl, as defined above; more preferably, R₅ is selected from the group consisting of oleyl, octyl, myristoleyl, palmitoleyl and sapienyl; still more preferably, R₅ is selected from oleyl and octyl; even more preferably R₅ is oleyl.

X is selected from the group consisting of O and S, preferably X is O.

In a preferred embodiment, the glycoside derivative of formula (I) is selected from the group consisting of:
- oleyl 2-acetamido-2-deoxy-α-D-glucopyranoside;
- oleyl 2-acetamido-2-deoxy-β-D-glucopyranoside;
- oleyl 2-deoxy-2-trifluoroacetamido-α-D-glucopyranoside;
- oleyl 2-deoxy-2-trifluoroacetamido-β-D-glucopyranoside;
- oleyl 2-acetamido-2-deoxy-6-O-(oxosulfonyl)-α-D-glucopyranoside potassium salt;
- oleyl 2-acetamido-2-deoxy-6-O-(oxosulfonyl)-β-D-glucopyranoside potassium salt;
- oleyl 2-amino-2-deoxy-α-D-glucopyranoside;
- oleyl 2-amino-2-deoxy-β**-**D-glucopyranoside;
- octyl 2-acetamido-2-deoxy-6-O-[2,2-bis(hydroxymethyl)-3-hydroxypropyl]-α-D-glucopyranoside;
- octyl 2-acetamido-2-deoxy-6-O-[2,2-bis(hydroxymethyl)-3-hydroxypropyl]-β-D-glucopyranoside; and
- any combination thereof.

In a particular embodiment, the glycoside derivative of formula (I) is selected from the group consisting of:
- oleyl 2-acetamido-2-deoxy-α-D-glucopyranoside;
- oleyl 2-deoxy-2-trifluoroacetamido-α-D-glucopyranoside;
- oleyl 2-acetamido-2-deoxy-6-O-(oxosulfonyl)-α-D-glucopyranoside potassium salt;
- oleyl 2-amino-2-deoxy-α-D-glucopyranoside;
- octyl 2-acetamido-2-deoxy-6-O-[2,2-bis(hydroxymethyl)-3-hydroxypropyl]-α-D-glucopyranoside; and
- any combination thereof.

The glycoside derivatives of formula (I), as defined above, comprised in the coating of the micelle may be the same or different i.e. a mixture of 1, 2, 3, 4, or 5 glycoside derivatives of formula (I) as defined above, in any ratio. In a particular embodiment, the glycoside derivatives of formula (I) are the same.

The term "hydrodynamic size" of the micelle refers to the diameter of the nanoparticles plus the surfactant and the glycoside derivative (or coating).

The hydrodynamic size is determined for nanoparticles or micelles in suspension in an electrolyte. In said suspension, the ions of the electrolyte are distributed around the surface of the nanoparticle or micelle. A first monolayer of ions of the opposing sign electrostatically bound to the nanoparticle or micelle and a surrounding area of enveloping solution that has an excess of ions having the sign opposite that of the nanoparticle or micelle, outside this area the concentration of ions being constant at all points and equal to that of the electrolyte. The diameter of the monolayer and the enveloping area is the hydrodynamic size. The size is measured in 10 mM phosphate buffer at 25°C, pH 7.1, in a Malvern Zetasizer nanoZS.

In a preferred embodiment of the invention, the zeta potential of the defined micelles of the present invention is in the range from -100 mV to 100 mV, preferably from -100 mV to -5mV and from 5 mV to 100 mV, still more preferably from -100 mV to -20 mV and from 20 mV to 100 mV, still more preferably from -100 mV to - 50 mV and from 50 mV to 100 mV.

The term "zeta potential" or ζ refers to a particle surface charge measurement. The zeta potential is determined for micelles in suspension in an electrolyte. In said suspension, the ions of the electrolyte are distributed around the surface of the micelle. A first monolayer of ions of the opposing sign electrostatically bound to the micelle and a surrounding area of enveloping solution having an excess of ions having the sign opposite that of the micelle, outside this area the concentration of ions being constant at all points and equal to that of the electrolyte. The difference in potential between the monolayer and the enveloping area is the zeta potential. The zeta potential determines the degree of repulsion between adjacent micelles having a charge of the same sign. If it drops by more than a specific value, the attractive forces exceed the repulsive forces, and the micelle aggregate. The potential was measured in solutions of the micelles with 0.01 M KNO₃, measuring the electrophoretic mobility in a Malvern Zetasizer nanoZS.

In a preferred embodiment of the invention, the previously defined micelles comprising nanoparticles having an iron oxide core have a transversal relaxivity in the range from 50 mM⁻¹s⁻¹ to 300 mM⁻¹s⁻¹, preferably in the range from 150 mM⁻¹s⁻¹ to 300 mM⁻¹s⁻¹.

In a particular embodiment of the invention, the previously defined micelles have a longitudinal relaxivity in the range of 0.5 mM⁻¹s⁻¹ to 5 mM⁻¹s⁻¹, preferably in the range from 3 mM⁻¹s⁻¹ to 5 mM⁻¹s⁻¹.

The term "transversal relaxivity" or r₂ and the term "longitudinal relaxivity" or r₁ refer to a measurement of the capability of micelles to decrease the transversal relaxation time (T₂) and longitudinal relaxation time (T₁), respectively, of the surrounding protons per unit of iron concentration. This capability is relevant in designing contrast agents for diagnostic imaging techniques such as magnetic resonance imaging (MRI). MRI uses the magnetic spins of hydrogen nuclei aligned by an external magnetic field. A radio frequency pulse disrupts the alignment of spins in equilibrium, relaxation of the spin back to equilibrium is monitored at a high temporal resolution. Two relaxation processes take place, longitudinal relaxation (T₁) and transversal relaxation (T₂). The preceding relaxation processes can be monitored independently to generate different magnetic resonance images. Local variations in the proton spin density, largely attributed to water molecules and caused by changes in the biological environment, affect the relaxation responses from which images can be constructed. Iron oxide nanoparticles affect the transverse relaxation time (T₂) of water. These relaxivity values vary depending on micelle size.

The transversal and longitudinal relaxivities are determined by using a Bruker minispec mq60 relaxometer with a field of 1.5 T.

In a particular embodiment, the micelles are monodisperse micelles.

The term "monodisperse" refers to micelles having a PDI (polydispersivity index) value equal to or less than 0.30, preferably equal or less to 0.25, more preferably equal or less to 0.20, i.e., they have substantially the same hydrodynamic size, wherein at least 70% of the micelles have the same size, preferably at least 75%, more preferably at least 80%, respectively. A variation of ± 30% of the defined hydrodynamic size, preferably ± 25%, more preferably ± 20%, must be understood as "substantially the same hydrodynamic size".

In a preferred embodiment, in the micelle according to the present invention, the concentration (in mg/mL) of the glycoside derivative of formula (I) to the concentration (in mg/mL) of the main metal comprising in the nanoparticles core (in the examples it is iron) is in the range of 1:1 to 50:1, preferably from 3:1 to 50:1, more preferably from 5:1 to 50:1, still more preferably from 10:1 to 5:1.

### Process for the preparation of micelles comprising nanoparticles

In another aspect, the present invention relates to a process for the preparation of a micelle as defined above which comprises:
a) providing one or more nanoparticles comprising a core and an outer layer as defined in above;
b) providing glycoside derivatives of formula (I) as defined above; and
c) mixing the nanoparticles of step a) and the glycoside derivatives of step b) under conditions adequate for the formation of hydrophobic interactions between the first member of the hydrophobic interaction pair of the outer layer of the nanoparticles and the R₅ groups in the glycoside derivatives.

The first step of the process, step a), consists of providing one or more nanoparticles comprising a core and an outer layer according to the present invention. The core of the nanoparticles can be iron oxide, UCNP and metals, as previously defined; preferably the core is an iron oxide core, as previously defined, still more preferably an iron oxide core selected from Fe₃O₄ and γ-Fe₂O₃.The outer layer of the nanoparticles comprises the first member of a hydrophobic interaction pair, as defined above.

The nanoparticles having an iron oxide core and an outer layer, as previously defined, used in step a) can be obtained by methods known by the person skilled in the art and described in the state of the art. An example of such methods is the co-precipitation in aqueous medium, wherein ferric and ferrous ions are mixed in highly basic solutions at room temperature or at a high temperature, said co-precipitation can be performed in the presence of a protective agent or surfactant or the nanoparticles obtained can subsequently be coated with said protective agent or surfactant. Another usual method for obtaining iron oxide nanoparticles is thermal decomposition of organic iron compounds, such as iron *N*-nitrosophenylhydroxylamine (Fe(cup)₃), iron acetylacetonate (Fe(acac)₃), iron pentacarbonyl (Fe(CO)₅), iron trichloride (FeCl₃), iron oleate, and sodium oleate/iron trichloride, at a high temperature. Thermal decomposition is generally performed in the presence of a surfactant. Another usual method for synthesizing iron oxide nanoparticles is sonochemical synthesis, wherein the iron precursors are broken down by the action of a high frequency (for example 20 KHz to 10 MHz).

The nanoparticles having a UCNP core and an outer layer, as previously defined, used in step a) can be obtained by methods known by the person skilled in the art and described in the state of the art. An example of such methods is the hydro(solvo)thermal synthesis in aqueous medium, wherein rare-earth precursors and fluoride precursors are mixed in high pressurized and high temperature autoclave. Said rare-earth precursors can be nitrates, oxides, chlorides and, more often, fluorines. Said fluoride precursors can be HF, NH₄F, NH₄HF₂, NaBF₄, KBF₄, NaF, KF, etc. Another usual method for obtaining UCNP nanoparticles is thermal decomposition procedure where the metal trifluoroacetates are thermally decomposed generally in the presence of a surfactant mixture (oleic acid, octadecene, trioctylphosphine, etc). Organic lanthanide chloro-precursors can be as well used in this method in combination with a fluoride source such as NaF. Other methods investigates preparation of UCNP nanoparticles by phase transfer liquid, solid and solution phases ie LSS method, microwave irradiation, etc.

Addition of inorganic layer of the same material without the dopant (as described above) around the first core, is usually achieved mimicking the same method used in presence of the single core nanoparticles. Thus a thin inorganic layer of the non-doped material is added around the core of the nanoparticles.

The nanoparticles having a metal core and an outer layer, as previously defined, used in step a) can be obtained by methods known by the person skilled in the art and described in the state of the art.

Gold or silver nanoparticles can be obtained by hydrothermal synthesis from an aqueous solution of the appropriate Au or Ag compound, trisodium citrate, and cetyltrimethylammonium bromide (CTAB) surfactant. By heating the mixture at 110 °C for 6, 12, 24, 48, and 72 h, particles with approximate average sizes of 30, 60, 90, 120, and 150 nm can be obtained

Preferably, the nanoparticles having a metal core and an outer layer provided in step a) are dispersed in an apolar solvent such as n-pentane, cyclopentane, n-hexane, cyclohexane, cyclohexane, n-heptane, chloroform, diethyl ether, benzene, toluene, 1,4-dioxane, dichloromethane, and mixtures thereof. The solvent is preferably n-hexane.

The second step of the process, step b), consists of providing glycoside derivatives of formula (I) according to the present invention. Said glycoside derivatives of formula (I) are known in the art and may be obtained by synthetic procedures such as those disclosed in García-Álvarez I. et al. [J. Med. Chem. 2007, 50, 364-373; J. Med. Chem., 2011, 54, 6949-6955], Fernández-Mayoralas A. et al. [J. Med. Chem. 2003, 46, 1286-1288] and co-pending Spanish patent application P201230510.

Compounds of formula (IV), i.e. glycoside derivatives of formula (I) wherein X is O, R₁ is H, C₁-C₆ alkyl optionally substituted with one or more substituents selected from the group consisting of C₁-C₃ alkyl, hydroxyl-C₁-C₃ alkyl, and hydroxyl; C₁-C₆ acyl, R₂-R₃ and R₅ are as defined above, R₄ is C₁-C₃ acyl, may be obtained by reaction of a compound of formula (II), wherein R₁-R₃ are H or C₁-C₃ acyl, R₄ is C₁-C₃ acyl and LG is a leaving group such as halogen and hydroxyl, with a compound of formula (III) wherein R₅ is as defined above in the presence of an acid, such as SnCl₄ (Scheme 1).

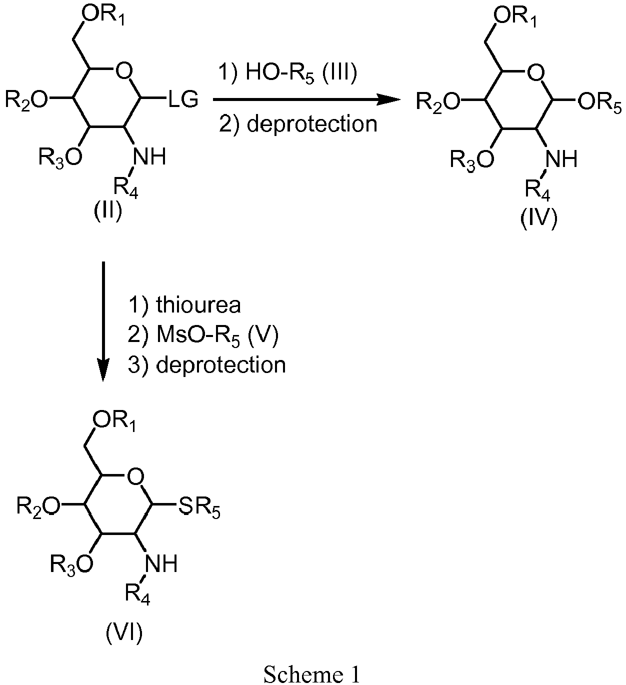

Compounds of formula (VI), i.e. glycoside derivatives of formula (I) wherein X is S, R₁ is H, C₁-C₆ alkyl optionally substituted with one or more substituents selected from the group consisting of C₁-C₃ alkyl, hydroxyl-C₁-C₃ alkyl, and hydroxyl; C₁-C₆ acyl, R₂-R₃ and R₅ are as defined above, R₄ is C₁-C₃ acyl, may be obtained by reaction of a compound of formula (II), wherein R₁-R₃ are H, R₄ is C₁-C₃ acyl and LG is a leaving group such as halogen and hydroxyl, with thiourea followed by reaction with a compound of formula (V) wherein R₅ is as defined above in the presence of a base, such as triethylamine (Scheme 1). If R₁-R₃ are C₁-C₃ acyl in the compound of formula (II), a compound of formula (IV) or (VI) wherein R₁-R₃ are H may be obtained by deprotection of the acyl groups by methods known in the art, such as reaction in basic media such as NaOMe/MeOH. If R₁-R₃ are H in the compound of formula (II), a compound of formula (IV) or (VI) wherein R₁ is C₁-C₃ acyl and R₂-R₃ are C₁-C₆ acyl may be obtained by acylation by methods known in the art, such as reaction of the corresponding acyl chloride or acid anhydride in pyridine in the presence of 4-dimethylaminopyridine.

The above compounds of formula (IV) and (VI) wherein R₁-R₃ are H, R₄ is C₁-C₃ acyl and R₅ is as defined above, may be deacylated by methods known in the art, such as treatment with basic media to render the corresponding compounds wherein R₄ is H.

Glycoside derivatives of formula (I) having a SO₃M group at R₁ may be obtained by reaction of compounds of formula (IV) or (VI) wherein R₁-R₃ are H, R₄ is C₁-C₃ acyl and R₅ is as defined above, with butane-2,3-dione followed by sulfation with SO₃-pyridine complex and acid hydrolysis.

The SO₃M alkali salt may be obtained by addition of the corresponding alkali hydroxide to the glycoside derivative of formula (I) wherein R₁ is SO₃H, typically in a polar solvent such as a polar protic solvent, for example, methanol, ethanol, n-propanol, isopropanol, n-butanol and water, or a polar aprotic solvent, for example tetrahydrofuran, acetone, dimethylformamide (DMF), acetonitrilo, dimethylsulfoxide (DMSO), and mixtures thereof.

Alternatively, the SO₃M alkali salt may be obtained by eluting an aqueous solution of the glycoside derivative of formula (I) wherein R₁ is SO₃H, through ion exchange resin previously activated with an alkali solution.

The SO₃M ammonium or quaternary amine salts may be obtained by addition of an aqueous solution of the corresponding ammonium or quaternary amine salts and then evaporated to completely dryness by freeze-drying to obtain the corresponding salt.

Step b) in the process for the preparation of the micelle consists of providing glycoside derivatives of formula (I) according to the present invention.

Preferably, the glycoside derivatives of formula (I) provided in step b) are dispersed in a polar solvent such as a polar protic solvent, for example, methanol, ethanol, n-propanol, isopropanol, n-butanol and water, or a polar aprotic solvent, for example dichloromethane, tetrahydrofuran, ethyl acetate, acetone, dimethylformamide (DMF), acetonitrilo, dimethylsulfoxide (DMSO), and mixtures thereof. The solvent is preferably an aqueous solution. The term "aqueous solution" refers to a solution in which the solvent is water or mostly water, i.e., wherein at least 55% of the volume of the solvent (water) with respect to the total aqueous solution volume is water, preferably at least 60%, more preferably at least 70%, more preferably at least 80%, more preferably at least 90%, even more preferably at least 95%, even more preferably at least 99%. The solvent of said aqueous solution comprises water as the major component but it can further comprise other water-miscible solvents, such as alcohols, for example, methanol, ethanol, isopropanol, ethers, such as for example tetrahydrofuran, 2-methyltetrahydrofuran, dioxane and mixtures thereof. The solvent of the aqueous solution is preferably water, and may further contain acids, bases or salts dissolved in the solvent.

The third step of the process, step c), consists of mixing the nanoparticles of step a) and the glycoside derivatives of step b) under conditions adequate for the formation of hydrophobic interactions between the first member of the hydrophobic interaction pair of the outer layer of the nanoparticles and the R₅ groups in the glycoside derivatives

"Adequate conditions" for the formation of the hydrophobic interactions are known for the skilled in the art, such as reverse evaporation methods, infusion methods or nanoemulsion methods, preferably nanoemulsion methods. Briefly, evaporation method consists in mixing the hydrophobic nanoparticles (having an outer layer comprising the firs member of the hydrophobic interaction pair) and the glycoside derivatives of formula (I) in organic solvent (most of the time chloroform or dichloromethane). After slow controlled evaporation of the organic solvent, the thin mixture film is re-dispersed in an aqueous medium. Examples of adequate conditions for nanoemulsion methods is mixing of an aqueous phase containing the amphiphilic glycoside derivatives of formula (I) described above with an organic phase containing the hydrophobic nanoparticles, preferably in a 10:1 volume proportion, although other proportions may be used. Upon sonication for 15 min to 90 min at a temperature comprised between 30 °C and 60 °C, an oil in water nanoemulsion is formed which turns progressively in one aqueous homogenous phase by evaporation of the organic solvent (usually hexane).

The micelles obtained in step c) may be purified by conventional methods known by the person skilled in the art, such as filtration for removing aggregates, centrifugation and redispersion or size exclusion by chromatography column.

In a particular embodiment, in the process of the invention the nanoparticles are provided in an apolar organic solvent, the glycoside derivatives are provided as a dispersion in an aqueous solution and step c) comprises generating an oil in water emulsion and further removing organic solvents from the emulsion.

### Pharmaceutical uses

Glycoside derivatives of formula (I) show anticancer activity, in particular in glioma, lung carcinoma and melanoma, as described in García-Álvarez I. et al. [J. Med. Chem. 2007, 50, 364-373; J. Med. Chem., 2011, 54, 6949-6955] and co-pending Spanish patent application P201230510, and are thus useful in the treatment of cancer.

The micelles comprising nanoparticles according to the present invention, which comprise glycoside derivatives of formula (I), have also shown anticancer activity.

Thus, another aspect relates to a micelle comprising nanoparticles according to the present invention for use in medicine.

In another aspect, the invention relates to the use of a micelle according to the present invention for the manufacture of a medicament.

A further aspect relates to a micelle comprising nanoparticles according to the present invention for use in the treatment and/or prevention of cancer, preferably cancer selected from lung cancer and glioma.

Another aspect relates to the use of a micelle comprising nanoparticles according to the present invention in the manufacture of a medicament for the treatment and/or prevention of cancer, preferably cancer selected from lung cancer and glioma.

Another aspect relates to a method of treatment and/or prevention of cancer, preferably, cancer selected from lung cancer and glioma, comprising administering to a subject in need thereof a therapeutically effective amount of the micelle comprising nanoparticles according to the present invention.

The term "treatment" is used to designate the administration of a micelle according to the invention or a medicinal product containing it to control the progression of the disease before or after the clinical signs have appeared. Control of the progression of the disease is understood as the beneficial or desired clinical results which include but are not limited to reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied.

"Prevention" is understood as the administration of a micelle according to the invention or of a medicinal product containing it in an initial or early stage of the disease, or to also prevent its onset.

"Medicinal product" is understood as a pharmaceutical composition comprising a micelle according to the invention. Said pharmaceutical composition may comprise pharmaceutically acceptable excipients, such as vehicles, diluents or adjuvants. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The terms "cancer" and "tumor" relate to the physiological condition in mammals characterized by unregulated cell growth. The micelles according to the present invention are useful for the treatment of any cancer or tumor, such as, without limitation, lung, breast, heart, small intestine, colon, splenic, kidney, bladder, head, neck, ovarian, prostate, brain (such as glioma), pancreatic, skin, bone, bone marrow, blood, thymic, uterine, testicular and liver tumors.

In the sense used herein, "therapeutically effective amount" refers to the amount of active ingredient calculated to produce the desired effect, and it will generally be determined, among others, by the characteristics of the active ingredient used and the therapeutic effect that will be obtained.

Micelles of the invention may be administered by the parenteral, oral, sublingual, subcutaneous, transdermal, intranasal, intraocular, and/or rectal routes, preferably by parenteral route.

The following examples represent specific embodiments of the present invention. They do not intend to limit in any way the scope of the invention defined in the present description.

### Examples

### Nanoparticle and micelle characterization methods

The size and shape of the particles was studied using a 200-KeV JEOL-2000 FXII electron transmission microscope. A drop of a diluted suspension of the micelles in PBS 1X was placed on a copper grid, coated with carbon and dried at 50°C.

The hydrodynamic diameter was characterized by means of measuring dynamic light scattering using a Nano Sizer ZS (Malvern) and nanoparticles dispersions at a concentration of 5 mM Fe in PBS 1X.

The particles (0.5 mM Fe) were diluted in a 0.01 M solution of KNO₃ to measure the zeta potential. HNO₃ or KOH may be used for the pH variations when measuring the zeta potential.

The samples were analyzed by Fourier transform infrared spectroscopy (FTIR) using Perkin Elmer 400 equipment for the direct measurement of powder samples.

A MINISPEC MQ60 (Bruker) at 37°C with a magnetic field of 1.5 T was used to evaluate the performance of the particles as contrast agents in resonance imaging. The relaxation rates Rᵢ (1/Tᵢ, s⁻¹, i = 1, 2) were obtained from the direct measurement of relaxation times (Tᵢ, s). The linear fit of data, Rᵢ vs. concentration of Fe or Gd, allows obtaining relaxivity values for different nanoparticles (rᵢ, s⁻¹mM⁻¹).

For the determination of iron content, 100 µl of glyconanoparticles solution was first hydrolyzed overnight with 1 ml of an HCl / HNO₃ (1:1) solution. The resultant mixture containing the hydrolyzed iron ions was diluted to 25 ml. Finally, inductively coupled plasma atomic emission spectroscopy (ICP-AES) was performed and concentration of iron determined by comparison with a calibration curve. ICP-MS analysis were carried out in a ICP PERKIN ELMER mod. OPTIMA 2100 DV with a elemental analizer CNHS PERKIN ELMER 2400 with the hydrolyzed under standard conditions after the hydrolysis of the nanoparticles with hydrogen peroxide/ nitric acid mixtures.

For the determination of the glycoside concentration, 1 ml of micelle aqueous solution was lyophilized in order to remove the H₂O. The crude extract was re-suspended in a mixture of H₂O-EtOH (preferently 1:2, 1.5 ml) and placed under sonication at room temperature for 10 min. After that, the solution was centrifuged 30 min at 5000 rpm, 4°C and the superrnatant containing the free glycoside derivative of formula (I) was analyzed by HPLC. Precipitate was re-suspended again and the same operation was repeated to ensure complete extraction of the glycoside. The comparison with a calibration curve performed with known concentrations of the free glycoside gave the final glycoside concentration in the glyconanoparticles solution. All HPLC analyses were performed using a Jasco Pu-2089 Plus pump equipped with an ultraviolet Jasco UV-2075 Plus detector and a 20 ml Rheodyne injector. Data acquisition and processing were accomplished with the Jasco ChromPass Chromatography Data System 1.8.6.1 software. The flow rate was 1.0 ml min⁻¹ and the elution profile was monitored by recording the UV absorbance at 205 nm. A reverse phase Licrosorb C18 (5 µm, 4.6 x 250 mm) column was used as stationary phase and the mobile phase was 100 % MeOH. For glycosides containing any sulfate group, a normal phase Kromasil-NH₂ (5 µm, 4.6 x 250 mm) column was used as stationary phase and glycoside separation was carried out with a mobile phase made with 40% of H₂O and 60% of solvent B (solvent B is a 50:50 mixture of acetonitrile and 20 mM sodium phosphate buffer, pH 5.6).

### Glycoside derivatives characterization methods.

Melting points are not corrected and were measured with a Reicher Jung Thermovar micro-melting apparatus.

Optical rotations were recorded on a Perkin Elmer 241 Polarimeter (λ=589 nm, 1 dm cell).

¹H NMR spectra were registered at 400 or 300 MHz and ¹³C NMR spectra were obtained at 100 MHz on a Varian INOVA spectrometers, using CDCl₃, CD₃OD or D₂O as solvent at room temperature. Chemical shift values are reported in parts per million (δ). Coupling constant values (J) are reported in hertz (Hz), and spin multiplicities are indicated by the following symbol: s (singlet), d (doublet), t (triplet), q (quartet), m (multiplet).

High resolution mass spectra (HRMS) were recorded on an Agilent 6520 Accurate Mass Q-TOF spectrometer with an ESI source.

### Example 1: Preparation of oleic acid coated ultra-small super paramagnetic iron oxide nanoparticles

The nanoparticles were synthesized using iron acetylacetonate as precursor and phenyl ether as the solvent. A mixture of Fe(acac)₃ (0.71 g, 2 mmol), 1,2-hexadecanediol (2.38 g, 10 mmol), oleic acid (1.69 g, 6 mmol), oleylamine (1.6 g, 6 mmol) and phenyl ether (20 mL) were mixed in a three-neck flask. Then, the reaction mixture was heated under mechanical stirring and a flow of nitrogen gas until a temperature of 200 °C was reached. This temperature was kept for 120 min and then the solution was heated to reflux (254 °C) for 30 min under nitrogen. Subsequently, the solution was cooled to room temperature. To remove the side products, ethanol was added to the reaction mixture and the resulting solution was centrifuged at 8500 rpm for 10 min. The supernatant was decanted; hexane (20 mL) and oleic acid (0.05 mL) were added to the nanoparticles and the suspension was centrifuged as 8500 rpm in order to remove aggregates and to obtain a stable suspension.

### Example 2: Synthesis of glycoside derivatives of formula (I)

The following glycoside derivatives of formula (I) were synthesized:

**Table 1**

| Name | Structure | Synthetic procedure |
|---|---|---|
| Oleyl 2-acetamido-2-deoxy-α-D-glucopyranoside (GC22) | 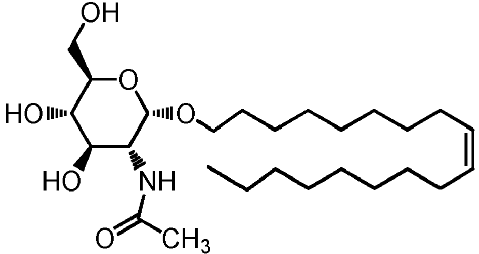 | Described in García-Álvarez I. et al., J. Med. Chem. 2011, 54, 6949-6955 |
| Oleyl2-deoxy-2-trifluoroacetamido-α-D-glucopyranoside (TFA-GC22) | 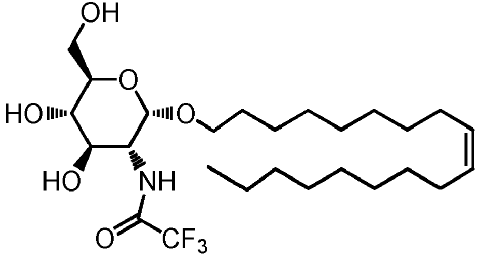 | Described below and in co-pending Spanish patent application P201230510] |
| Oleyl2-amino-2-deoxy-α-D-glucopyranoside (GC22-NH₂) | 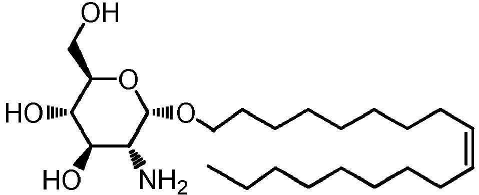 | Described below |
| Oleyl 2-acetamido-2-deoxy-6-*O-*(oxosulfonyl)-α-D-glucopyranoside potassium salt (IG20) | 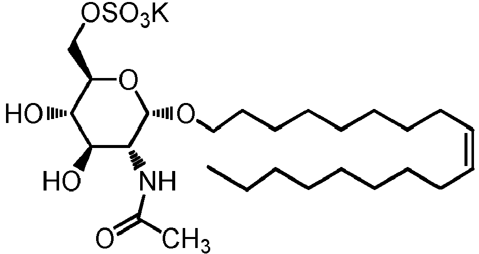 | Described in García-Álvarez I. et al., J. Med. Chem. 2007, 50, 364-373 |
| Octyl 2-acetamido-2-deoxy-6-*O*-[2,2-bis(hydroxymethyl)-3-hydroxypropyl]-α-D-glucopyranoside (NF115) | 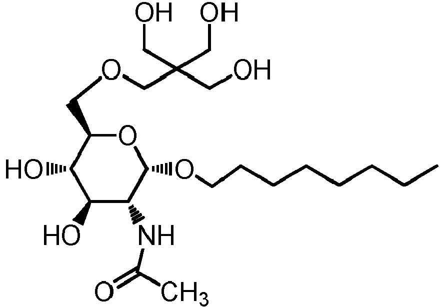 | Described in Fernández-Mayoralas A. et al., J. Med. Chem. 2003, 46, 1286-1288 |

### Synthesis of oleyl 2-deoxy-2-trifluoroacetamido-α-D-glucopyranoside (TFA-GC22).

*N*-trifluoroacetyl-D-glucosamine was prepared as described in the literature (Wolfrom, M. L. and Conigliaro, P. J. (1969) Trifluoroacetyl as an N-protective group in the synthesis of purine nucleosides of 2-amino-2-deoxy saccharides, Carbohydr Res 11, 63-76).

*N*-trifluoroacetyl-D-glucosamine (1.18 g, 4.29 mmol) was dissolved in oleic alcohol (85%) (8 ml, 21.4 mmol) and was treated with H₂SO₄-silica (35%) (236 mg). The reaction mixture was stirred under argon at 180 °C for 20 min (TLC: EtOAc). After this time, the mixture was cooled at room temperature and purified by silica gel column chromatography (hexane-EtOAc, 2:1→0:1) to give TFA-GC22 (604 mg, 29%) as a white solid. Mp: 118-123 °C. [α]_{D}: +86.0° (c 0.5, MeOH). ¹H NMR (400 MHz, CD₃OD): δ 5.4-5.3 (m, 2H), 4.86 (d, 1H, *J*=3.6 Hz), 3.90 (dd, 1H, *J*=10.8, 3.6 Hz), 3.9-3.8 (m, 1H), 3.80 (t, 1H, *J*=1.9 Hz), 3.8-3.7 (m, 2H), 3.60 (ddd, 1H, *J*=9.9, 5.5, 2.3 Hz), 3.5-3.3 (m, 2H), 2.1-1.9 (m, 4H), 1.8-1.5 (m, 2H), 1.5-1.1 (m, 22H), 0.90 (t, 3H, *J*=7.0 Hz). ¹³C RMN (100 MHz, CD₃OD): δ 159.2, (q, *J*=37.5 Hz), 117.5 (q, *J*=287.6 Hz), 130.8, 130.8, 97.7, 73.8, 72.3, 71.9, 68.98, 62.6, 56.2, 33.1, 30.9, 30.9, 30.6, 30.6, 30.6, 30.5, 30.5, 30.4, 30.3, 28.1, 28.1, 27.3, 23.8, 14.5 ppm. HRMS (ESI+) *m*/*z* (calcd 525.3283): 526.3345 (M-H)⁺, 543.3621 (M-NH₄)⁺. HRMS (ESI+) m/z (calcd 525.3283): 526.3345 (M-H)⁺, 543.3621 (M-NH₄)⁺.

For preparation of H₂SO₄-silica catalyst (35%), silica gel (200-325 mesh) (10 g) was resuspended in ethylic ether (50 mL) and concentrated H₂SO₄ (3 mL) was slowly added drop by drop with stirring. During the absorption process, 10 min, the suspension was gently stirred at room temperature. After this time, the solvent was removed by distillation and the silica was dried under reduced pressure (2-4 mbar) and heated at 60 °C for 3 h.

### Synthesis of oleyl 2-amino-2-deoxy-α-D-glucopyranoside (GC22-NH₂)

TFA-GC22 (Oleyl 2-deoxy-2-trifluoroacetamido-α-D-glucopyranoside) (0.2 g, 0.38 mmol) was resuspended in a solution (5 mL) containing CH₃OH, H₂O and Et₃N (72%:10%:18%). The reaction was incubated under argon at 70 °C for 48 h. (TLC: EtOAc-MeOH, 10:1). Then, the solution was evaporated to a solid residue and the residue was purified by silica gel column chromatography (EtOAc-MeOH, 10:1) to give the compound GC22-NH2 as a yellow oil (149 mg, 92%).

¹H NMR (300 MHz, CD₃OD) δ 5.47 - 5.44 (m, 2H), 4.79 (d, *J*=3.6 Hz, 1H), 3.97 (dd,1H), 3.8 (m, 2H), 3.62 (m, *J*=10.0 Hz), 3.37 (t, 1H), 3.35 (dt, 1H), 3.08 (t, 1H), 2.69 (dd, *J =* 10.0, 3.6 Hz, 1H), 2.16 - 1.83 (m, 4H), 1.62 (m, 2H), 1.51 - 1.15 (m, 22H), 0.90 (t, *J=* 5.3 Hz, 3H). MS (+ESI) m/z (calcd. C₂₄H₄₇NO₅: 429.3454): 430.3551 (M+H)+.

### Example 3: Preparation of the micelle comprising the glycoside derivatives of formula (I)

The glycoside derivative of formula (I) obtained in Example 2 (10 to 20 mg) in ethanol (1 mL) was first dispersed in 15 mL of phosphate buffer (pH=7.2, 5 mM). 1 mL of the suspension comprising oleic acid coated iron oxide nanoparticles (NP) in hexane obtained in Example 1 (10 to 15 mg/mL) was then added to the solution and the resultant mixture was sonicated (Branson 250, 42 +/- 6 KHz) under robust stirring for 20 min at 37 °C. Oil in water (o/w) nanoemulsion was further kept under sonication for 1 hour to evaporate all traces of hexane and ethanol resulting in the formation of a homogenous micellar aqueous solution. Aggregates were removed after filtration (0.22 µm, PVDF membrane).

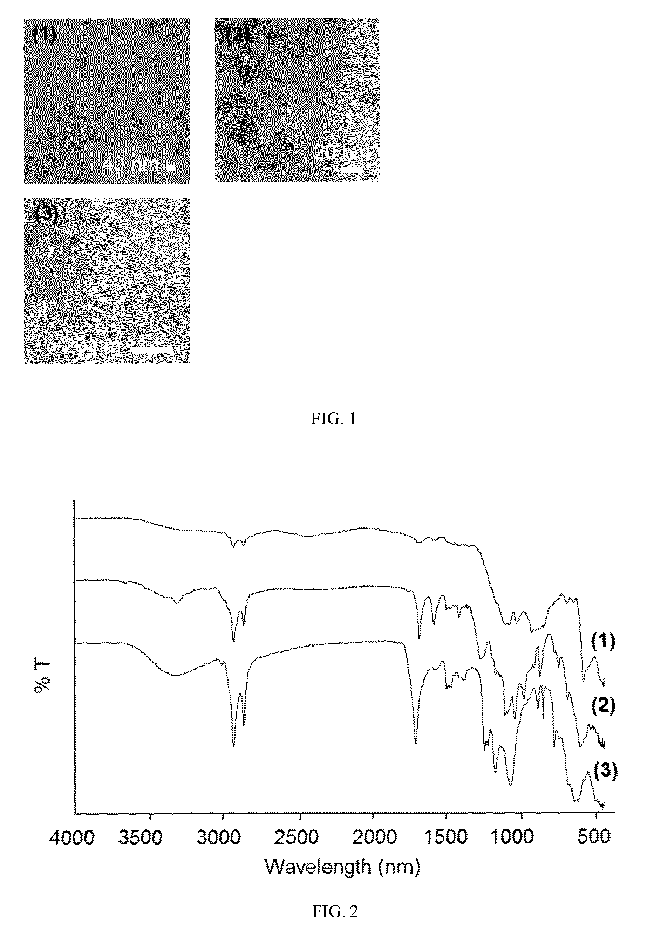

The obtained micelles were characterized using the procedures described above and the results are shown in Table 2 and Figures 1 and 2.

**Table 2. Characterization of micelles**

| Micelle | Glycoside derivative of formula (I) | Size (nm) | Pdi | Zeta potential (mV) | [Fe³⁺] mg.ml⁻¹ | [glycoside] mg.ml⁻¹ | Relaxometry (s⁻¹.Mm⁻¹) |
|---|---|---|---|---|---|---|---|
| GC22-NP | GC22 | 61.8 | 0.24 | -16 | 0.5 | ∼ 5 | r1 2,8/ r2 184 |
| TFAGC2 2-NP | TFAGC22 | 65.8 | 0.18 | -44 | 1.1 | ∼ 2.7 | r1 3,8/ r2 186 |
| IG20-NP | IG20 | 32.3 | 0.27 | +47 | 0.3 | ∼ 2.5 | r1 3,6/ r2 137 |

### Example 4: In vitro activity of the micelles, inhibition of A549 and C6 tumor cell proliferation

Human A549 cell line and C6 rat glioma cell line were maintained in Dulbecco's modified Eagle's medium (DMEM) complete medium (Sigma-Aldrich, St. Louis, MO), supplemented with fetal bovine serum (FBS, 10%; GLinus, Madrid, Spain), glutamine (2 mM), penicillin (50 IU/mL), and streptomycin (50 mg/mL), at 37 °C in a 5% CO₂ humidified atmosphere. Exponentially growing cells were seeded on 96-well plates (Beckton Dickinson, Le Pont de Claix, France), in DMEM complete medium, at a density of 1.5 x 10⁴ cells/well for C6 cell and 5 x 10³ cells/well for A549 cells respectively. The cells were allowed to attach with 5% of CO₂ at 37°C for overnight, and then, to serum starve the cells, the medium was replaced by 100 µL of fresh DMEM without FBS and maintained overnight. Stock solutions in EtOH of the glycoside derivatives of formula (I) of Example 2 (50 mM) or stock solutions in PBS of the micelles of Example 3 were finally dissolved in DMEM complete medium for the treatments. The cells were treated with serial dilutions of glycoside derivatives of formula (I) of Example 2 or micelles of Example 3 for 48 h. For the experiments with micelles of Example 3, some 96-well plates were placed over a MagnetoFACTOR-96 device (Chemicell) to produce a strong magnetic field to each well and increase the proximity of the nanoparticles to the cells. Also, iron oxide nanoparticles coated with azelaic acid were tested (Azelaic-NP, obtained as described in Chemistry - A European Journal, 2008, 14 (30), 9126-9130) Cell proliferation was evaluated with an MTT assay (Sigma-Aldrich), based on the conversion of the water-soluble MTT into an insoluble formazan. Briefly, after 48 h of compound treatment, the medium was replaced by 100 µL of fresh DMEM without phenol red containing MTT (5 mg/mL) solution, and cells were incubated for additional 3 h at 37 °C with 5% CO₂. After this time, the medium was removed, the precipitated formazan was dissolved in 100 µL of DMSO, and the solution optical density was measured at 595 nm in Spectramax Plus equipment (Molecular Devices Corporation). Absorption values were referred to positive proliferation controls (cells cultured in DMEM complete medium) and negative proliferation controls cells cultured in DMEM without FBS). Inhibition was expressed as an ID₅₀ value, the compound concentration that reduced maximal proliferation by 50%, and the results are shown in Table 3.

**Table 3. Biological activity of glycoside derivatives of formula (I) and micelles comprising said glycoside derivatives.**

| Compound | ID₅₀ (µM) A549 | ID₅₀ (µM) C6 |
|---|---|---|
| GC22 | 10 | 15.5±0.3 |
| TFAGC22 | 8.6 | 14.2±0.3 |
| IG20 | 97 | >100 |
| GC22-NP | 100.5 w/o magnet | 55 w/o magnet |
| | 95 with magnet | 70 with magnet |
| TFAGC22-NP | 40.3 w/o magnet | 24.4 w/o magnet |
| | 42 with magnet | 49.8 with magnet |
| IG20-NP | 64.4 w/o magnet | 68.5 w/o magnet |
| | 91 with magnet | 57.2 with magnet |

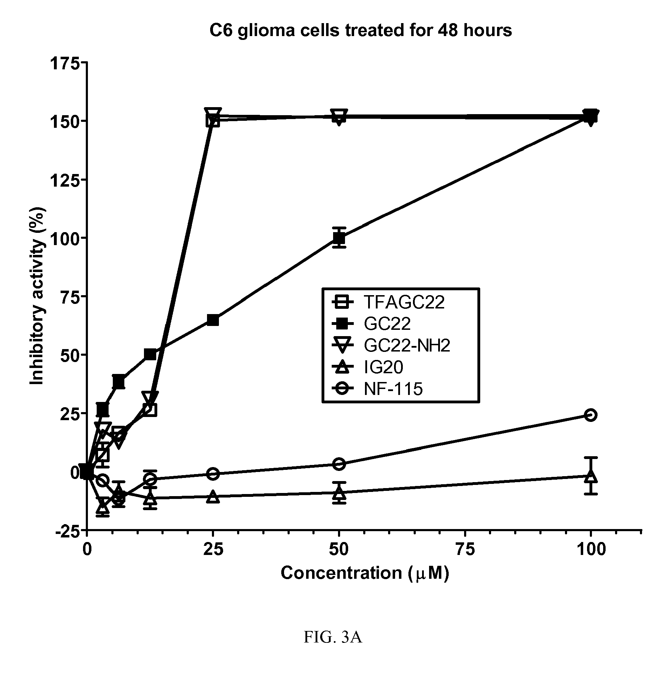

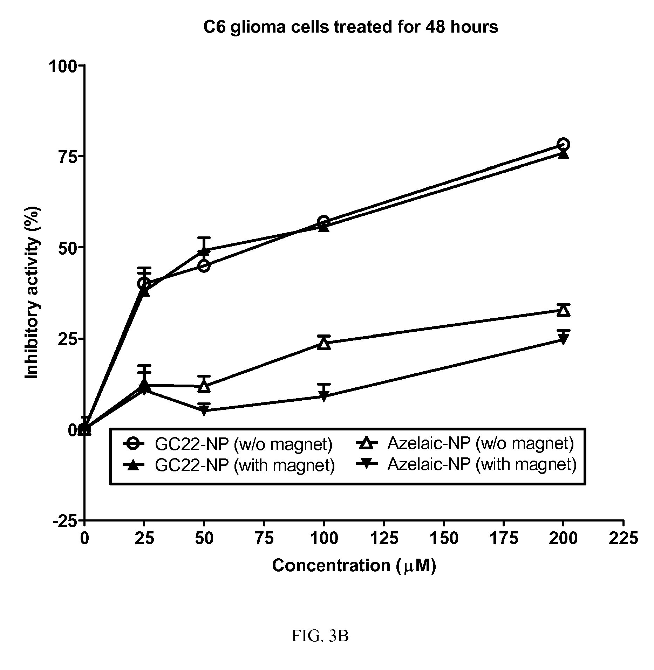

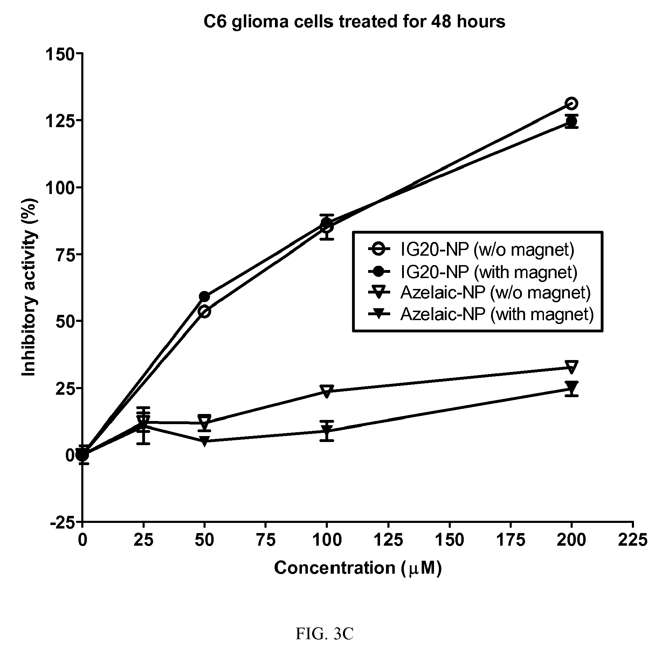

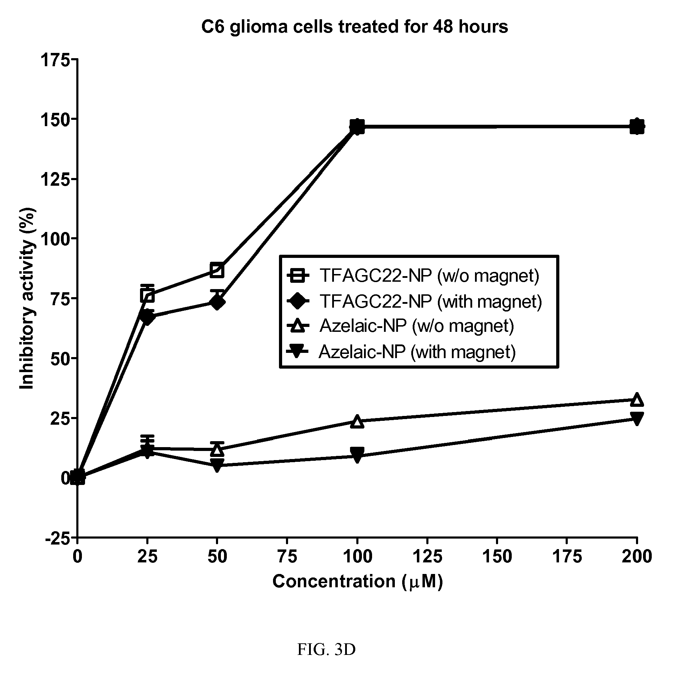

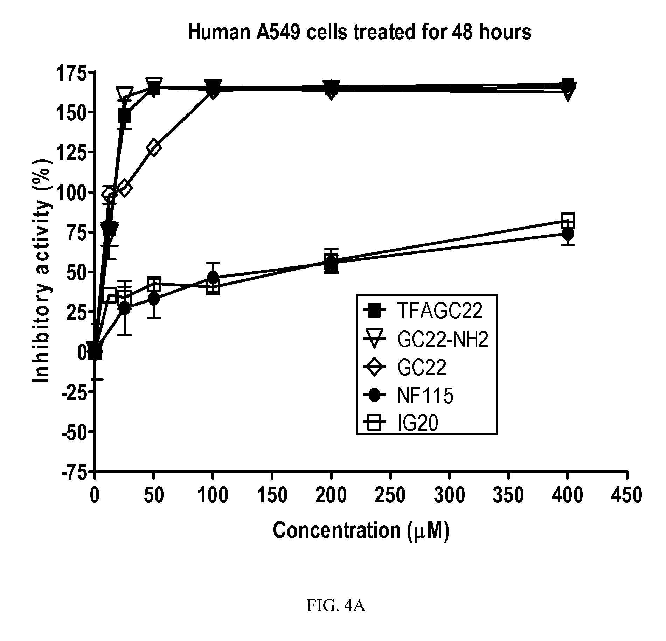

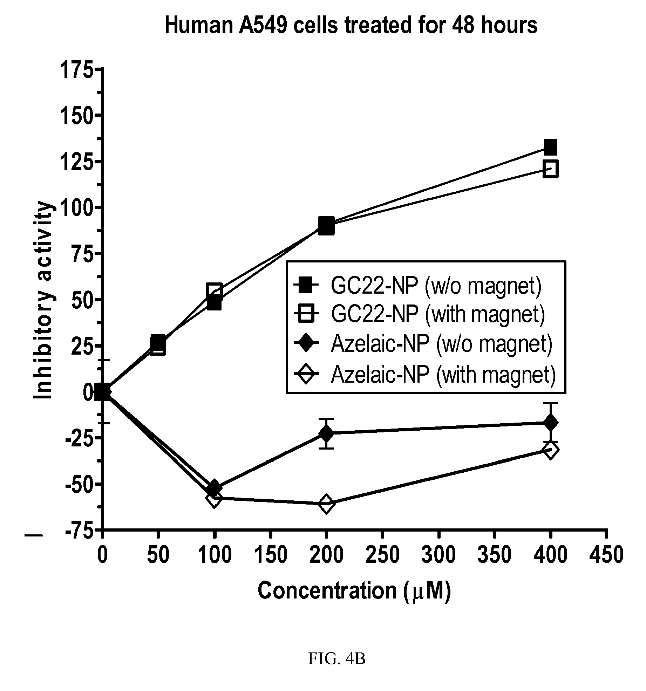

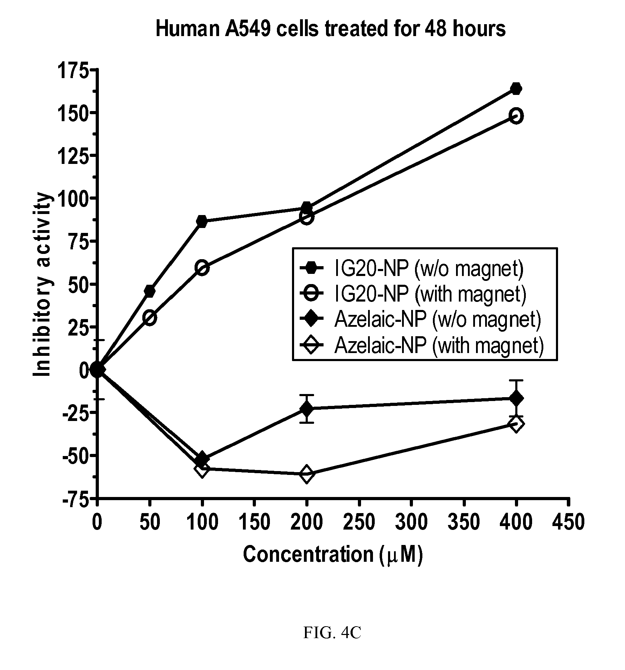

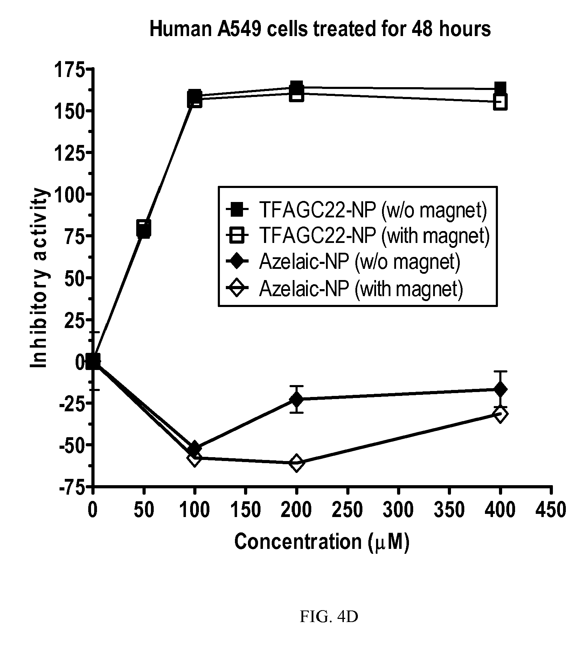

Inhibitory activities of the single glycosides with respect to their molar concentration against C6 glioma and A549 alveolar tumor cell lines are shown respectively in Figure 3A and Figure 4A. Inhibitory activities of the micelles with respect to their concentration against C6 glioma tumor cell line are shown in Figures 3B-3D. Inhibitory activities of the micelles with respect to their molar concentration against A549 alveolar tumoral cell line are shown in Figures 4B-4D. As it can be seen, micelles of Example 3 (GC22-NP, TFAGC22-NP or IG20-NP) inhibit proliferation of A549 and C6 tumor cells. Surprisingly, in the case of IG20-NP micelles are inhibitors or tumor cell proliferation in C6 cells, whereas glycoside IG20 *per se* did not show inhibition of C6 cell proliferation. Moreover, micelles IG20-NP are more active as inhibitors or tumor cell proliferation in A549 cells than glycoside IG20 *per se.* It can also be seen that when the biological activity is determined in the presence of magnetic field the results are worse. This maybe because the magnet attracts straight away the NPs in the center of the plate thus they cannot disperse in all the plate where there is the cell culture.

### Example 5: GC22-NP kinetic of elimination in rat's liver after intravenous administration (i.v.a)

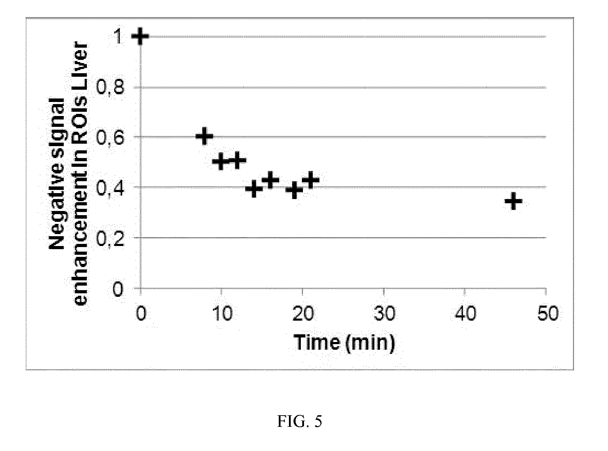

Rat (n = 1, Wistar, 350 g) was anesthetized with 2% isoflurane in a mixture of N₂/O₂ (80:20). Baseline images were acquired before the intravenous administration of 300 µl, [Fe]=1.5 mg.ml⁻¹ of micelles comprising glycoside derivative GC22 obtained in Example 3 (GC22-NP). Magnetic resonance images of the rat's liver were acquired at different times, from 5 minutes up to 1 hour after the injection. Specific region of interests (ROI) were selected and the reduction of the signal measured. After normalization to the baseline, curve representing the kinetic of elimination of the liver was represented (Figure 5).

Magnetic Resonance Imaging (MRI) protocol. The images were acquired with a 7 T Biospec MRI spectrometer (Bruker, Ettlingen, Germany), using a transmitter/receiver quadrature coil of 25 mm inner diameter (Bruker, Ettlingen, Germany). Rat was kept anaesthetized with 2% isoflurane in a mixture of N₂/O₂ (80:20) via facial mask and placed prone in a custom-built plastic holder. The body temperature was kept constant using warm circulating water and the respiratory cycle was monitored constantly. 8 axial slices of 1 mm thickness were acquired to image the liver. The acquisition was performed in free-breathing animals, using a FLASH sequence.

### Example 6: Solubility

For GC-22 and TFA-GC22, the glycosides alone are not soluble in pure water, while the micelles GC-22-NPs and TFA-GC22-NPs are stable in the aqueous phase. Precipitate of GC-22 and TFA-GC22 can be observed by eye straight away in a pure water solution and no re-dispersion is produced when the aqueous micellar solution is mixed with an organic solvent selected from the group consisting of dichloromethane, chloroform, hexane, heptane, toluene, in a volume proportion of 1:1. Only IG-20 is soluble in water (but in the case of this one, bioactivity is increased so this is other advantage in this case).

## Claims

1. Micelle comprising:
a) one or more nanoparticles having a core selected from the group consisting of iron oxide, UCNP and metals, and an outer layer comprising a first member of a hydrophobic interaction pair; and
b) a coating comprising glycoside derivatives of formula (I) 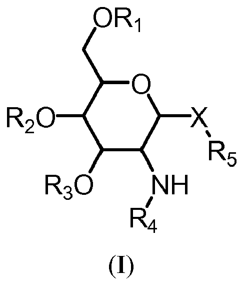 wherein
R₁ is selected from the group consisting of H; C₁-C₆ alkyl optionally substituted with one or more substituents independently selected from the group consisting of C₁-C₃ alkyl, hydroxyl-C₁-C₃ alkyl, and hydroxyl; C₁-C₆ acyl; and SO₃M wherein M is selected from the group consisting of hydrogen, alkali metal, ammonium and quaternary amine;
R₂ and R₃ are independently selected from the group consisting of H and C₁-C₆ acyl;
R₄ is selected from the group consisting of H and C₁-C₃ acyl optionally substituted with one or more halogen atoms;
R₅ is a second member of the hydrophobic interaction pair; and
X is selected from the group consisting of O and S;
or a stereoisomer thereof;
wherein the coating is associated to the nanoparticle by means of hydrophobic interactions between the first member of the hydrophobic interaction pair of the outer layer of the nanoparticles and the R₅ groups in the coating.

2. Micelle according to claim 1, wherein the nanoparticle core is iron oxide.

3. Micelle according to claim 2, wherein the iron oxide is selected from the group consisting of Fe₃O₄ and γ-Fe₂O₃.

4. Micelle according to any preceding claim, wherein the first and second members of the hydrophobic interaction pairs are the same or different.

5. Micelle according to any preceding claim wherein the first member of the hydrophobic interaction pair is a fatty acid and/or the second member of the hydrophobic interaction pair is a fatty acid radical.

6. Micelle according to any preceding claim, wherein the first member of the hydrophobic interaction pair is oleic acid and R₅ is selected from oleyl or octyl.

7. Micelle according to any preceding claim, wherein the one or more nanoparticles have a size in the range of 5 nm to 200 nm.

8. Micelle according to any preceding claim, wherein R₁ is selected from the group consisting of H, SO₃M wherein M is selected from the group consisting of hydrogen, alkali metal, ammonium and quaternary amine, and C₁-C₃ alkyl optionally substituted with one or more substituents independently selected from the group consisting of hydroxyl-C₁-C₃ alkyl and hydroxyl.

9. Micelle according to any preceding claim, wherein R₂ and R₃ are H.

10. Micelle according to any preceding claim, wherein R₄ is selected from H, acetyl and trifluoroacetyl.

11. Micelle according to any preceding claim, wherein X is O.

12. Micelle according to any preceding claim, wherein the glycoside derivative of formula (I) is selected from the group consisting of:
- oleyl 2-acetamido-2-deoxy-α-D-glucopyranoside;
- oleyl 2-acetamido-2-deoxy-β-D-glucopyranoside;
- oleyl 2-deoxy-2-trifluoroacetamido-α-D-glucopyranoside;
- oleyl 2-deoxy-2-trifluoroacetamido-β-D-glucopyranoside;
- oleyl 2-acetamido-2-deoxy-6-O-(oxosulfonyl)-α-D-glucopyranoside potassium salt;
- oleyl 2-acetamido-2-deoxy-6-O-(oxosulfonyl)-β-D-glucopyranoside potassium salt;
- oleyl 2-amino-2-deoxi-α-D-glucopyranoside;
- oleyl 2-amino-2-deoxi-β-D-glucopyranoside;
- octyl 2-acetamido-2-deoxi-6-O-[2,2-bis(hydroxymethyl)-3-hydroxypropyl]-α-D-glucopyranoside;
- octyl 2-acetamido-2-deoxi-6-O-[2,2-bis(hydroxymethyl)-3-hydroxypropyl]-β-D-glucopyranoside; and
- any combination thereof.

13. Micelle according to any preceding claim, wherein the ratio of glycoside derivative of formula (I) to nanoparticles is in the range of 1:1 to 20:1 (w/w).

14. Process for the preparation of a micelle as defined in any preceding claim which comprises:
a) providing one or more nanoparticles comprising a core and an outer layer as defined in any of claims 1 to 7;
b) providing glycoside derivatives of formula (I) as defined in any of claims 1 and 6 to 12; and
c) mixing the nanoparticles of step a) and the glycoside derivatives of step b) under conditions adequate for the formation of hydrophobic interactions between the first member of the hydrophobic interaction pair of the outer layer of the nanoparticles and the R₅ groups in the glycoside derivatives.

15. The process according to claim 14 wherein the nanoparticles are provided in an apolar organic solvent, wherein the glycoside derivatives are provided as a dispersion in an aqueous solution and wherein step c) comprises generating an oil in water emulsion and further removing organic solvents from the emulsion.

16. Micelle as defined in any of claims 1 to 13 for use in medicine.

17. Micelle as defined in any of claims 1 to 13 for use in the treatment and/or prevention of cancer.

18. Micelle for use according to claim 17, wherein the cancer is selected from lung cancer and glioma.
